# EUROPEAN PATENT APPLICATION

(11) **EP 4 583 339 A1**
(43) Date of publication of application: **09.07.2025**
(21) Application number: 23859300.8
(22) Date of filing: 28.08.2023
(51) Int. Cl.: H02H 7/20, H02H 9/02, A24F 40/50

(54) **OUTPUT CONTROL CIRCUIT AND AEROSOL GENERATION APPARATUS**

(30) Priority: 29.08.2022 CN 202222289493 U; 24.08.2023 CN 202322298256 U
(71) Applicant: Changzhou Patent Electronic Technology Co., Ltd, Changzhou, Jiangsu 213000 (CN)
(72) Inventor: QIU, Weihua, Changzhou, Jiangsu 213000 (CN); CHEN, Hansen, Changzhou, Jiangsu 213000 (CN)
(74) Representative: Metida
(86) International application number: PCT/CN2023/115274
(87) International publication number: WO 2024/046270

(57) **Abstract**

The present invention belongs to the field of aerosol generation apparatus control, and particularly relates to an aerosol generation apparatus. The aerosol generation apparatus comprises a power source circuit, a switch circuit, a control circuit and a detection circuit, wherein an output end of the power source circuit is connected to a first input end of the control circuit for supplying power to the control circuit by means of the power source circuit; an output end of the detection circuit is connected to a second input end of the control circuit, and the detection circuit is used for generating an electrical signal according to a detected connection state of an atomizer and outputting the electrical signal to the control circuit, and an output end of the switch circuit is connected to a third input end of the control circuit for transmitting a switch signal to the control circuit, and the control circuit controls the operating state of the atomizer according to the switch signal. The present invention can determine a connection state of an atomizer by means of a control circuit and a detection circuit, thereby improving the usage experience of a user.

## Description

### TECHNICAL FIELD

The present invention relates to the control field of aerosol generation apparatus, and more specifically, to an aerosol generation apparatus.

### BACKGROUND

Existing aerosol generation devices, in order to increase the number of cycles for battery component reuse, typically employ a replaceable cartridge design. When the e-liquid in the cartridge is exhausted, a new cartridge can be installed. However, due to errors or defects in the production or assembly process of the cartridge, improper installation may cause the e-cigarette to malfunction. Therefore, after the user installs a new cartridge, there is a possibility that the e-cigarette will not work properly. The user, however, cannot directly determine the current status of the e-cigarette, which significantly affects the user experience.

Therefore, it is necessary to provide a new type of aerosol generation apparatus.

### SUMMARY OF THE DISCLOSURE

In view of the above-mentioned problems existing in the prior art, the purpose of this invention is to provide an aerosol generation apparatus that can determine the connection status of the atomizer through the control circuit and the detection circuit, thereby enhancing the user experience.

To achieve the above objectives, the technical solution adopted by the present invention is an aerosol generation apparatus, which includes a power supply circuit, a switch circuit, a control circuit, and a detection circuit, an output end of the power supply circuit is connected to a first input end of the control circuit for supplying power to the control circuit through the power supply circuit; an output end of the detection circuit is connected to a second input end of the control circuit, the detection circuit is configured to generate an electrical signal based on a detected connection status of an atomizer and output the electrical signal to the control circuit; an output end of the switch circuit is connected to a third input end of the control circuit to transmit a switch signal to the control circuit, the control circuit controls a working status of the atomizer based on the switch signal.

Further, the power supply circuit includes a battery and a charging circuit. The charging circuit is configured to supply power to the battery and includes at least one of USB, Type-C, charging terminal, or wireless charging.

Further, the power supply circuit includes a battery and a protection circuit. The protection circuit is used to protect the battery, with one end connected to the battery and the other end connected to the control circuit.

Further, the detection circuit includes a plug-in detection circuit, which is used to detect the connection status of the atomizer and includes a detection resistor R31.

Further, the detection circuit also includes a resistance detection circuit, which includes a MOSFET Q2 and a voltage divider resistor R25. The MOSFET Q2 is activated by the control signal from the control circuit to start the resistance detection circuit, with the voltage divider resistor R25 connected in series with the atomizer.

Further, the aerosol generation apparatus also includes a charging interface insertion detection circuit, which includes a diode D1.

Further, the aerosol generation apparatus also includes a load enhancement circuit, which includes a MOSFET Q1.

Further, the aerosol generation apparatus also includes a temperature control circuit, which is used to monitor the temperature of the circuit board and generate a temperature control signal output to the control circuit.

Further, the aerosol generation apparatus also includes a prompt circuit, the input end of which is connected to the output end of the control circuit, for indicating the working or connection status of the atomizer based on the control signal from the control circuit.

Further, the control circuit includes a main control chip, with the detection circuit integrated in the main control chip.

The beneficial effects of the present invention are as follows: The aerosol generation apparatus provided by the present invention includes a power supply circuit, a switch circuit, a control circuit, and a detection circuit; the output end of the power supply circuit is connected to the first input end of the control circuit to supply power to the control circuit through the power supply circuit; the output end of the detection circuit is connected to the second input end of the control circuit; the detection circuit is used to generate an electrical signal based on the connection status of the atomizer detected and to output the electrical signal to the control circuit; the output end of the switch circuit is connected to the third input end of the control circuit to transmit a switch signal to the control circuit, which controls the working status of the atomizer based on the switch signal. The present invention can determine the connection status of the atomizer through the control circuit and the detection circuit, thereby enhancing the user experience.

The present invention also proposes an output control circuit and an aerosol generation apparatus to improve the negative voltage generated when using PWM signals to output to the regulating circuit containing a MOSFET in existing technologies.

Basic technical solution: An output control circuit, which includes a protection circuit, an output circuit, a regulating circuit with a MOSFET, and a main control circuit with a main control chip;

the first input end of the main control circuit is connected to the first input end of the regulating circuit and transmits a PWM signal; the second output end of the main control circuit is connected to the first input end of the output circuit;

the first output end of the regulating circuit is connected to the second input end of the output circuit, and the second output end of the regulating circuit is connected to the input end of the protection circuit; the second output end of the protection circuit is connected to the second input end of the regulating circuit.

Beneficial effects of the basic solution: In this case, a protection circuit is set on both sides of the regulating circuit containing a MOSFET to reduce the load current generated by the junction capacitance of the MOSFET during the instant when the PWM signal is turned off. This can achieve a reduction/elimination of negative pressure and overshoot spikes in the regulating circuit containing a MOSFET, making the overall control circuit more stable and improving the safety of the downstream circuits of the control circuit.

Further, the first output end of the protection circuit is connected to the third input end of the output circuit.

Further, the protection circuit includes a first protection branch;

the second output end of the MOSFET serves as the second output end of the regulating circuit and is connected to the input end of the first protection branch; the output end of the first protection branch serves as the second output end of the protection circuit and is connected to the second input end of the MOSFET, which serves as the second input end of the regulating circuit; the first protection branch includes a first capacitor, one end of which serves as the input end of the first protection branch, and the other end serves as the output end of the first protection branch.

Further, the protection circuit includes a second protection branch with a first protection resistor; the second protection branch is connected in series between the first output end of the regulating circuit and the second input end of the output circuit; one end of the first protection resistor is connected to the second output end of the regulating circuit, and the other end is connected to the second input end of the output circuit.

Further, the protection circuit also includes a third protection branch, which includes a second protection resistor; one end of the second protection resistor is connected to both the input end of the regulating circuit and the output end of the main control circuit, and the other end is connected to the fourth input end of the output circuit, which is different from the first input end.

Further, the protection circuit also includes a fourth protection branch, which includes a current detection chip and a surface-mount MOSFET; the input end of the current detection chip is connected to the first output end of the regulating circuit, the output end of the current detection chip is connected to one end of the surface-mount MOSFET, and the other end of the surface-mount MOSFET is grounded.

Further, the second output end of the main control circuit is connected to the first input end of the output circuit, and the second output end of the main control circuit outputs another PWM signal.

Further, the second output end of the main control circuit is connected to one end of a second capacitor, and the other end of the second capacitor is grounded.

Further, the protection circuit also includes a third protection branch, which includes a second protection resistor. One end of the second protection resistor is connected to both the input end of the regulating circuit and the first input end of the main control circuit, and the other end is connected to the fourth input end of the output circuit and the output end of the first protection resistor.

The present invention also provides an aerosol generation apparatus, which includes the output control circuit described in any of the above items and an atomizing apparatus connected to the output end of the output circuit.

### BRIEF DESCRIPTION OF THE DRAWINGS

The following is a further description of the present invention in conjunction with the accompanying drawings and embodiments.

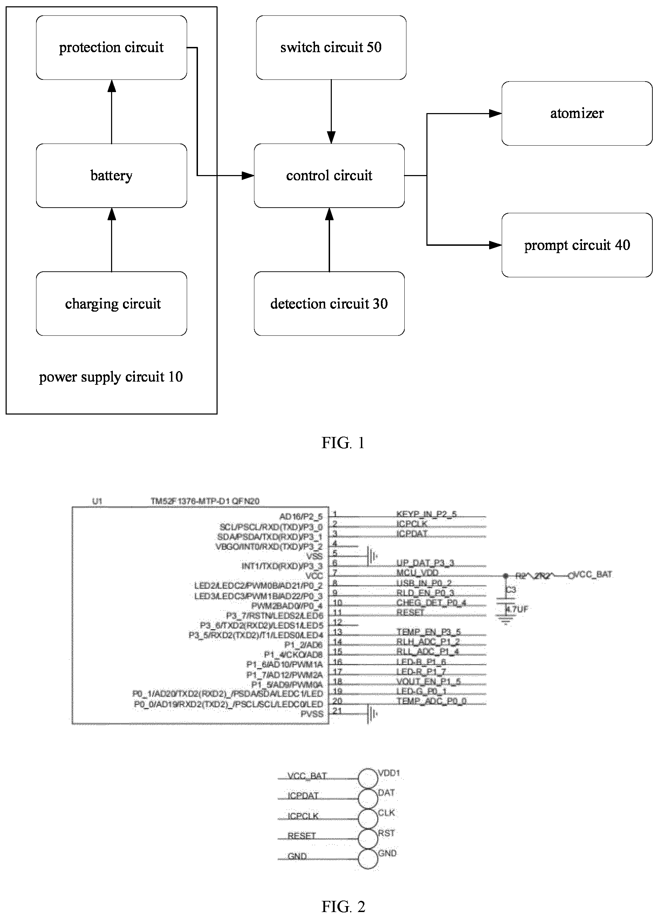

In the drawings: FIG. 1 is a structural diagram of the aerosol generation apparatus provided in Embodiment One of the present invention;
FIG. 2 is a circuit diagram of the control chip in the control circuit provided in Embodiment One of the present invention;
FIG. 3 is a circuit diagram of the switch circuit provided in Embodiment One of the present invention;
FIG. 4 is a circuit diagram of the temperature control circuit provided in Embodiment One of the present invention;
FIG. 5 is a circuit diagram of the charging circuit in the power supply circuit provided in Embodiment One of the present invention;
FIG. 6 is a circuit diagram of the protection circuit in the power supply circuit provided in Embodiment One of the present invention;
FIG. 7 is a circuit diagram of the detection circuit provided in Embodiment One of the present invention;
FIG. 8 is a circuit diagram of the prompt circuit provided in Embodiment One of the present invention;
FIG. 9 is a circuit diagram of the charging interface insertion detection circuit provided in Embodiment One of the present invention;
FIG. 10 is a structural diagram of the aerosol generation apparatus provided in Embodiment Two of the present invention;
FIG. 11 is a circuit diagram of the control chip in the control circuit provided in Embodiment Two of the present invention;
FIG. 12 is a circuit diagram of the button switch circuit in the control circuit provided in Embodiment Two of the present invention;
FIG. 13 is a circuit diagram of the charging circuit in the power supply circuit provided in Embodiment Two of the present invention;
FIG. 14 is a circuit diagram of the protection circuit in the power supply circuit provided in Embodiment Two of the present invention;
FIG. 15 is a circuit diagram of the prompt circuit provided in Embodiment Two of the present invention;
FIG. 16 is a circuit diagram of the load enhancement circuit provided in Embodiment Two of the present invention;
FIG. 17 is a structural diagram of an output control circuit provided in Embodiment Four of the present invention;
FIG. 18 is a structural diagram of the protection circuit in FIG. 17;
FIG. 19 is a structural diagram of the first protection branch in the protection circuit of FIG. 17;
FIG. 20 is a structural diagram of the first protection branch in the protection circuit of FIG. 17;
FIG. 21 is a structural diagram of the second protection branch in the protection circuit of FIG. 17;
FIG. 22 is a structural diagram of the third protection branch in the protection circuit of FIG. 17;
FIG. 23 is a circuit diagram of the main control chip in an output control circuit provided in Embodiment Five of the present invention;
FIG. 24 is a circuit diagram of the switch circuit in an output control circuit provided in Embodiment Five of the present invention;
FIG. 25 is a circuit diagram of the temperature control circuit in an output control circuit provided in Embodiment Five of the present invention;
FIG. 26 is a circuit diagram of the regulating circuit in an output control circuit provided in Embodiment Five of the present invention;
FIG. 27 is a circuit diagram of the protection circuit in an output control circuit provided in Embodiment Five of the present invention;
FIG. 28 is a circuit diagram of the protection circuit in an output control circuit provided in Embodiment Five of the present invention.

### DETAILED DESCRIPTION OF PREFERRED EMBODIMENTS

To further clarify the objectives, technical solutions, and advantages of the present invention, the various embodiments of the present invention will be described in detail below in conjunction with the accompanying drawings. However, those of ordinary skill in the art can understand that in the embodiments of the present invention, many technical details are proposed to better enable readers to understand the present application. Nevertheless, even without these technical details and various changes and modifications based on the following embodiments, the technical solutions claimed in the claims of the present application can still be realized.

### Embodiment One:

As shown in FIG. 1, Embodiment One of the present invention provides an aerosol generation apparatus, including a power supply circuit 10, a control circuit 20, a detection circuit 30, and a switch circuit 50.

The output end of the power supply circuit 10 is connected to the first input end of the control circuit 20 to supply power to the control circuit 20 through the power supply circuit 10. The output end of the detection circuit 30 is connected to the second input end of the control circuit 20. The detection circuit 30 is used to generate an electrical signal based on the connection status of the atomizer detected and to output the electrical signal to the control circuit 20. The output end of the switch circuit 50 is connected to the third input end of the control circuit 20 to transmit a switch signal to the control circuit 20, which controls the working status of the atomizer based on the switch signal. It should be noted that the electrical signal and switch signal include, but are not limited to, voltage signals, current signals, and level signals. Technical personnel may select according to actual needs, and the present application does not impose any limitations on this.

This embodiment can determine the connection status of the atomizer through the cooperation of the control circuit and the detection circuit. In actual use, when a cartridge containing the atomizer is inserted, the atomizer is in a connected and conductive state. When the cartridge is removed, the atomizer is in a disconnected state. This endows the aerosol generation apparatus with the function of detecting the connection status of the atomizer, thereby enhancing the user experience.

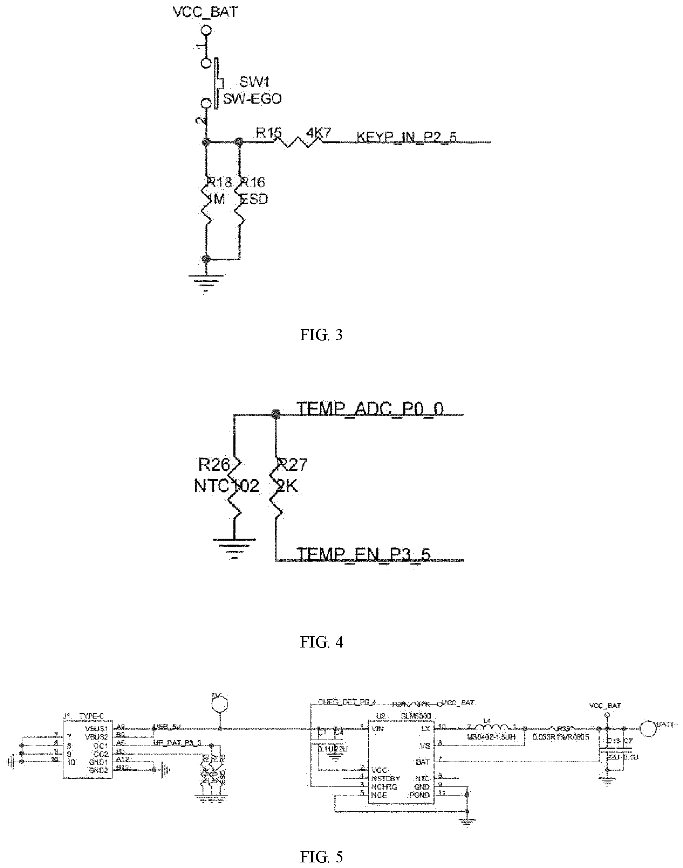

In this embodiment, the control circuit 20 includes a main control chip U1, which uses a TM52F1376-MTP-D1 model chip. The switch circuit 50 includes a button SW1 and a static resistor R16ESD. The temperature control circuit is used to monitor the temperature of the circuit board and includes a thermistor. Specifically, the circuit diagram of the main control chip U1 is shown in FIG. 2, the circuit diagram of the switch circuit 50 is shown in FIG. 3, and the circuit diagram of the temperature control circuit is shown in FIG. 4. The power supply VCC_BAT is connected in series with one branch of the button SW1's pin 1 and pin 2, connected to a fixed circuit R15, and then connected to pin 1 of the main control chip U1 to transmit the KEYP_IN_P2_5 signal. Another branch of pin 2 of the button SW1 is connected to a fixed resistor R18 and then grounded. The power supply supplies power to the main control chip U1 in the control circuit 20 through the switch circuit 50. In some embodiments, pin 20 TEMP_ADC_P0_0 of the main control chip U1 is connected to the temperature control circuit through a fixed resistor R27 and then connected to pin 13 TEMP_EN_P3_5 of the main control chip U1. Pin 20 TEMP_ADC_P0_0 of the main control chip U1 is also connected to one end of the thermistor R36, with the other end of the thermistor R36 grounded. By setting the thermistor R36, a temperature control signal can be generated and output to the control circuit 20. When the control circuit 20 receives a temperature control signal indicating that the circuit board temperature is too high, it controls the output of the power supply circuit 10 to be reduced or shut off to prevent damage to components due to excessive circuit board temperature, which could otherwise cause the aerosol generation apparatus to malfunction or even explode, severely affecting user experience and safety. It should be noted that a thermistor is used as the thermistor in this embodiment, but technical personnel may use other components capable of temperature monitoring functions, such as temperature sensors, according to actual needs. The present application does not impose any limitations on this.

In this embodiment, the power supply circuit 10 includes a battery and a charging circuit, which is used to supply power to the battery. The charging circuit includes at least one of USB, Type-C, charging terminal, or wireless charging. Preferably, the battery uses a lithium battery, but lead-acid batteries, nickel-cadmium batteries, nickel-iron batteries, nickel-metal hydride batteries, and other rechargeable batteries can also be used.

Specifically, as shown in FIG. 5, the charging circuit includes a USB J1 and a charging chip U2. The output end of the USB J1 is connected to the input end of the charging chip U2, and the output end of the charging chip U2 is connected to a lithium battery. The model of the USB J1 is TYPE-C, and the model of the charging chip U2 is SLM6300.

Specifically, pin A9 and pin B9 of the USB J1 are connected, and the USB_5V port of the USB J1 is connected to an external power supply. Pin A9 of the USB J1 is also connected to pin 1 of the charging chip U2 and transmits the USB_5V signal. Pin A5 of the USB J1 is connected to pin 6 of the control chip U1 to transmit the UP_DAT_P3_3 signal. Pins A12, B12, 7, 8, 9, 10, and 1 of the USB J1 are all grounded. Pins 5, 9, and 11 of the charging chip U2 are all grounded. Pin 10 of the charging chip U2 is connected to VCC_BAT through an inductor L4 and a resistor R35, i.e., the VCC_BAT output from the VCC end of the control chip U1. Pin 10 of the charging chip U2 is connected to the positive pole of the lithium battery through BATT+, and pin 8 of the charging chip U2 is connected to the right end of the inductor L4 through a fixed resistor R35 and then connected to VCC_BAT. Pin 8 of the charging chip U2 is also connected to the positive pole of the lithium battery through BATT+. Pin 3 of the charging chip U2 transmits the CHEG_DET_P0_4 signal, which is connected to VCC_BAT through a fixed resistor R34. The resistance value of the fixed resistor R34 is **47kΩ**.

In this embodiment, the power supply circuit also includes a battery and a protection circuit. The protection circuit is used to protect the battery, with one end connected to the battery and the other end connected to the control circuit. Preferably, the protection circuit includes a protection chip and a protection resistor, with the protection chip connected to the battery's electrode through the protection resistor. By setting the protection circuit, the battery can be effectively protected to prevent permanent and irreversible damage due to overcharging during charging and over-discharging during discharging.

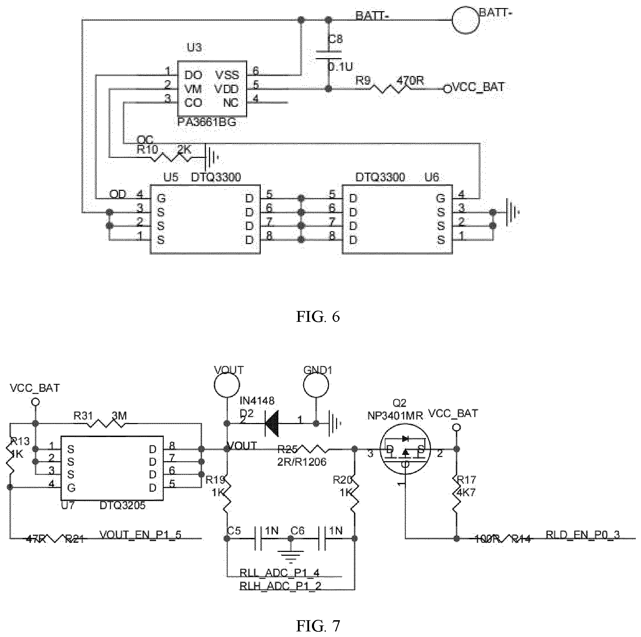

Specifically, as shown in FIG. 6, the protection circuit includes protection chips U3, U5, and U6. Protection chip U3 uses a PA3661BG model chip, and protection chips U5 and U6 both use a DTQ3300 model chip. Pins 5, 6, 7, and 8 of protection chips U5 and U6 are all interconnected. Pins 1, 2, and 3 of protection chip U5 are connected to the negative pole of the battery through BATT-, and pin 4 of protection chip U5 is connected to pin 1 of protection chip U3. Pins 1, 2, and 3 of protection chip U6 are all grounded, and pin 4 of protection chip U6 is connected to pin 3 of protection chip U3. Pin 3 of protection chip U3 is connected to a fixed resistor R10 and then grounded. Pin 6 of protection chip U3 is also connected to the negative pole of the battery through BATT-, and pin 5 of protection chip U3 is connected to VCC_BAT through a protection resistor R9. The resistance value of protection resistor R9 is 470Ω, and the resistance value of fixed resistor R10 is 2kΩ.

In this embodiment, the detection circuit 30 includes a plug-in detection circuit, which is used to detect the connection status of the atomizer. The plug-in detection circuit includes a detection resistor R31. The detection circuit 30 also includes a resistance detection circuit, which includes a MOSFET Q2 and a voltage divider resistor R25. The MOSFET Q2 is activated by the control signal from the control circuit 20 to start the resistance detection circuit, with the voltage divider resistor R25 connected in series with the atomizer.

Specifically, as shown in FIG. 7, the detection circuit 30 includes a chip U7, which uses a DTQ3205 model chip for the power output MOS of the lighter. Pins 1, 2, and 3 of chip U7 are all connected to VCC_BAT. Pin 4 of chip U7 is connected to VCC_BAT through a fixed resistor R13. Pin 4 of chip U7 is also connected to pin 18 of the main control chip U1 through a fixed resistor R21 to transmit the VOUT_EN_P1_5 signal. Pins 5, 6, 7, and 8 of chip U7 are all connected to VCC_BAT through the detection resistor R31, and pin 8 of chip U7 is also connected to the VOUT terminal. The VOUT terminal and GND1 terminal are the contact points of the heating element in the atomizer, with a diode D2 connected between the VOUT terminal and GND1 terminal. Specifically, the positive pole of the diode D2 is connected to GND1, and the negative pole is connected to the VOUT terminal. When the heating element in the atomizer is connected and conductive, the VOUT terminal is short-circuited, resulting in a low voltage. When the heating element in the atomizer is disconnected, due to the resistance value of the detection resistor R31 being 3MΩ, the power consumption is extremely low, and the voltage at the VOUT terminal is high. Therefore, the connection status of the atomizer can be determined by detecting the voltage at the VOUT terminal. Specifically, as shown in FIG. 7, the resistance detection circuit includes a field-effect transistor Q2 with a model number NP3401MR and a voltage divider resistor R25. The D end (pin 3) of the field-effect transistor Q2 is connected to one end of the voltage divider resistor R25 away from VOUT. The G end (pin 1) of the field-effect transistor Q2 is connected to pin 9 of the main control chip U1 through a fixed resistor R14 to receive the RLD_EN_P0_3 enable signal. That is, when the main control chip U1 sends a resistance detection signal through pin 9, the field-effect transistor Q2 is turned on to activate the resistance detection circuit. The voltage divider resistor R25 is connected in series with the heating element for voltage division. The voltage across the voltage divider resistor R25 is collected through pins 14 and 15 of the main control chip U1 to obtain the voltage and current in the circuit of the heating element, thereby measuring the resistance value of the heating element. By setting the resistance detection circuit, the status of the heating element can be effectively determined, allowing the control circuit 20 to adjust or shut off the output of the power supply circuit 10 in a timely manner based on the status of the heating element.

In this embodiment, the aerosol generation apparatus also includes a prompt circuit 40, the input end of which is connected to the output end of the control circuit 20 to provide prompts regarding the working or connection status of the atomizer based on the control signal from the control circuit 20.

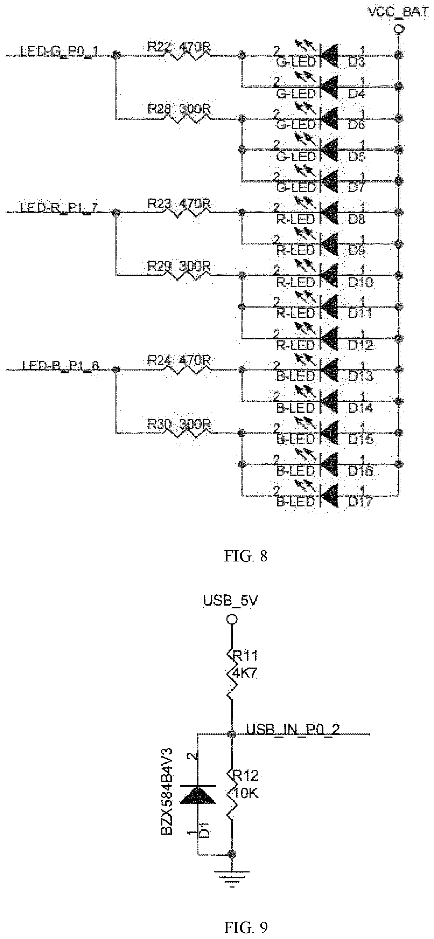

Specifically, as shown in FIG. 8, the prompt circuit 40 includes multiple parallel light-emitting diodes (LEDs), including three colors: green (G-LED), red (R-LED), and blue (B-LED). The positive terminals of the parallel LEDs are connected to VCC_BAT. The negative terminals of the green LEDs are connected to pin 19 of the main control chip U1 through a fixed resistor to transmit the LED-G_PO_1 signal. The negative terminals of the red LEDs are connected to pin 17 of the main control chip U1 through a fixed resistor to receive the LED-R_P1_7 signal. The negative terminals of the blue LEDs are connected to pin 16 of the main control chip U1 through a fixed resistor to receive the LED-B_P1_6 signal. The prompt circuit 40 is used to output corresponding prompt signals based on the instructions from the main control chip U1, thereby driving different LEDs to operate.

In this embodiment, the aerosol generation apparatus also includes a charging interface insertion detection circuit, which includes a diode D1. Specifically, as shown in FIG. 9, in the charging interface insertion detection circuit, the positive terminal of the diode D1 is grounded, and the negative terminal of the diode D1 is connected to the USB_5V signal of the USB J1 through a fixed resistor R11. The negative terminal of the diode D1 is also connected to pin 8 of the main control chip U1 to transmit the USB_IN_P0_2 signal. The charging interface insertion detection circuit presents different voltage values when the charging interface is inserted or not inserted, resulting in different USB_IN_P0_2 signals transmitted to pin 8 of the main control chip U1. Thus, the main control chip U1 can determine whether the charging interface has been inserted.

### Embodiment Two:

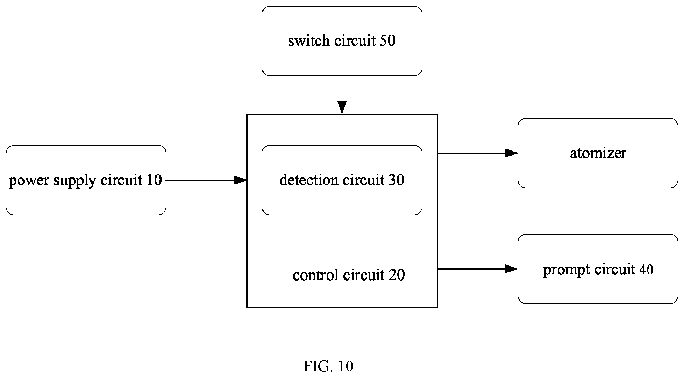

In this embodiment, as shown in FIG. 10, Embodiment Two of the present invention provides an aerosol generation apparatus, including a power supply circuit 10, a switch circuit 50, a control circuit 20, and a detection circuit 30.

The output end of the power supply circuit 10 is connected to the first input end of the control circuit 20 to supply power to the control circuit 20 through the power supply circuit 10. The control circuit 20 includes the detection circuit 30, which is used to generate an electrical signal based on the connection status of the atomizer detected and to output the electrical signal to the control circuit 20. The output end of the switch circuit 50 is connected to the third input end of the control circuit 20 to transmit a switch signal to the control circuit 20, which controls the working status of the atomizer based on the switch signal. It can be seen that the difference between Embodiment Two and Embodiment One lies in the fact that the detection circuit 30 in Embodiment Two is integrated into the control circuit.

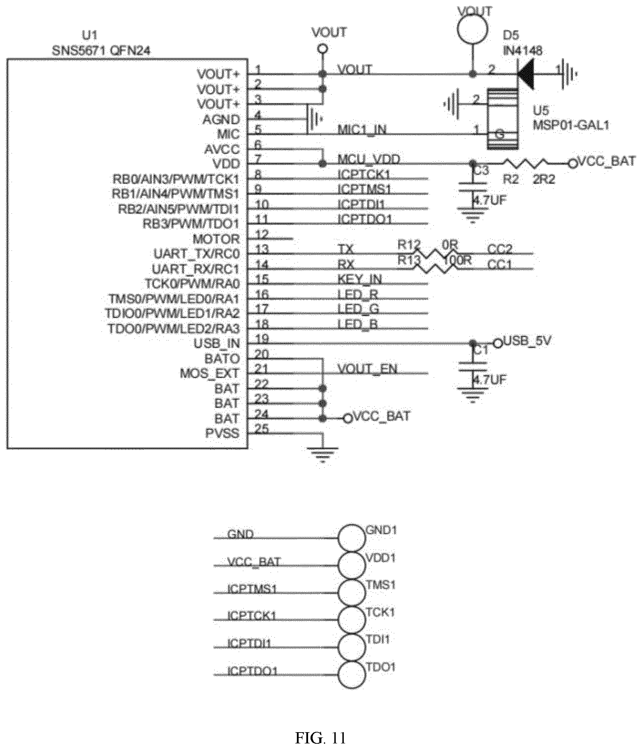

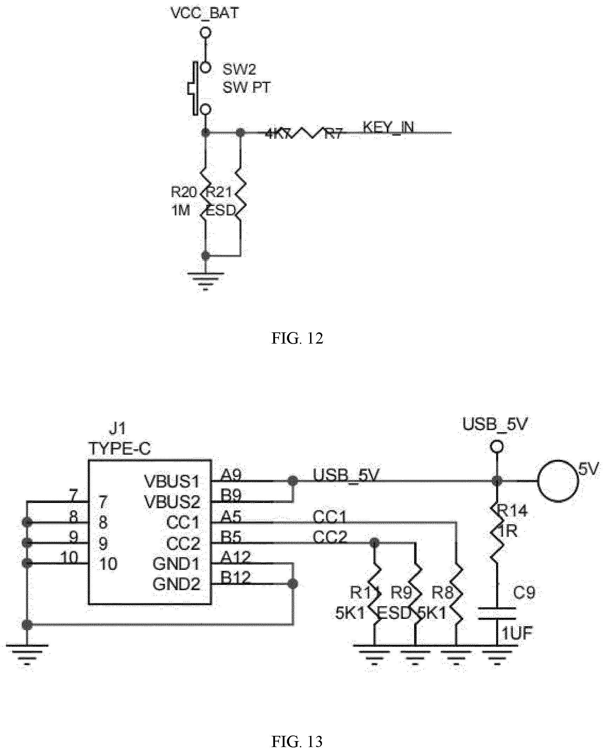

Specifically, as shown in FIG. 11, the control circuit 20 includes a main control chip U1, with the detection circuit 30 integrated into the main control chip U1. The detection circuit consists of a PGA and a CCO circuit, used for detecting the connection status of the atomizer in the cartridge containing the atomizer. Depending on the different states of insertion and disconnection of the cartridge, the voltage at the output end of the detection circuit 30 is different, i.e., the voltage detected by the main control chip U1 is different, thereby determining whether the cartridge is inserted or removed and outputting corresponding instructions. Pins 1-3 of the main control chip U1 are connected to VOUT. The switch circuit 50 includes an airflow sensor, which is connected to the main control chip U1. Pin 2 of the airflow sensor U5 is grounded, and pin 1 of the airflow sensor U5 is connected to pin 5 of the main control chip U1. The switch circuit 50 also includes a button switch circuit. Specifically, as shown in FIG. 12, the switch circuit includes a button SW2 and a static resistor R21ESD. The power supply VCC_BAT is connected in series with one branch of pin 1 and pin 2 of the button SW2, connected to pin 15 of the main control chip U1 through a fixed circuit R7 to transmit the KEYP_IN signal. Another branch of pin 2 of the button SW2 is connected to a fixed resistor R20 and then grounded. The power supply supplies power to the main control chip U1 in the control circuit 20 through the switch circuit 50.

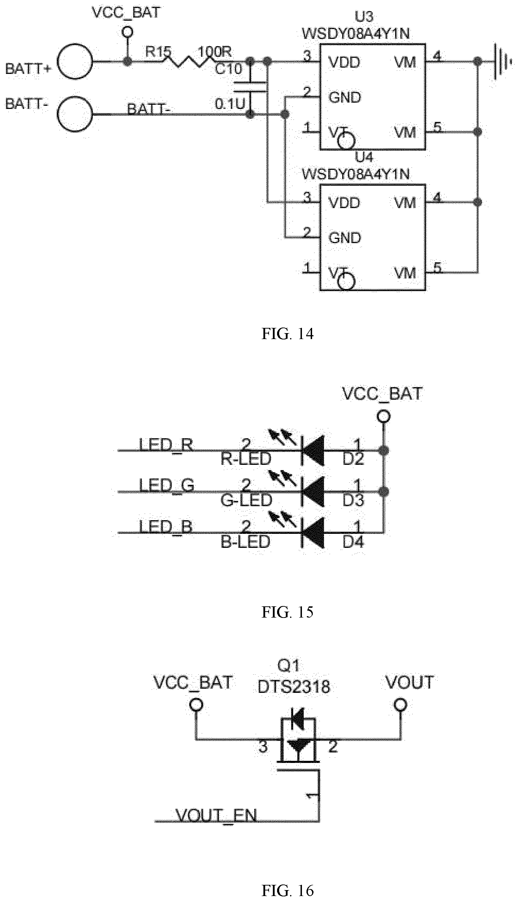

In this embodiment, the power supply circuit 10 includes a charging circuit and a protection circuit. The charging circuit is used to supply power to the battery, and the protection circuit is used to protect the battery from overcharging and over-discharging. As shown in FIG. 13, the charging circuit includes a USB J1 with a TYPE-C model. Pins A9 and B9 of the USB J1 are connected, and the USB_5V port of the USB J1 is connected to an external power supply. As shown in FIG. 14, the protection circuit includes chips U3 and U4, both of which use the WSDY08A4Y1N model chip. Pins 4 and 5 of chip U3 and pins 4 and 5 of chip U4 are connected and then grounded. Pins 2 of chip U3 and chip U4 are connected and then connected to the negative pole of the lithium battery through BATT-. Pins 3 of chip U3 and chip U4 are connected and then connected to the positive pole of the lithium battery through a fixed resistor R15 via BATT+.

As shown in FIG. 15, in this embodiment, the prompt circuit 40 includes multiple parallel light-emitting diodes (LEDs), including red (R-LED), green (G-LED), and blue (B-LED) LEDs. The positive terminals of the red, green, and blue LEDs are connected to VCC_BAT. The negative terminal of the red LED is connected to pin 16 of the main control chip U1 to transmit the LED_R signal. The negative terminal of the green LED is connected to pin 17 of the main control chip U1 to transmit the LED_G signal. The negative terminal of the blue LED is connected to pin 18 of the main control chip U1 to transmit the LED_B signal. The prompt circuit 40 is used to output corresponding prompt signals based on the instructions from the main control chip U1, thereby driving different LEDs to operate.

The aerosol generation apparatus also includes a load enhancement circuit, which includes a MOSFET Q1. Specifically, as shown in FIG. 16, the load enhancement circuit includes a MOSFET Q1. Pin 1 of the MOSFET Q1 is connected to pin 21 of the main control chip U1 to transmit the VOUT_EN signal. Pin 2 of the MOSFET Q1 is connected to VOUT, and pin 3 of the MOSFET Q1 is connected to VCC_BAT.

### Embodiment Three:

The aerosol generation apparatus provided in Embodiment Three is distinguished from that in Embodiment Two by the different model of the main control chip U1 in the control circuit 20. In this embodiment, the control circuit 20 includes a main control chip U1 using an LP7864 model chip. The main control chip U1 integrates the detection circuit 30 within its Power Control. The detection circuit 30 is used to detect the connection status of the atomizer in the cartridge containing the atomizer. Depending on the different states of insertion and disconnection of the atomizer in the cartridge, the voltage at the output end of the detection circuit 30 is different, i.e., the voltage detected by the main control chip U1 is different, thereby determining whether the cartridge is inserted or removed and outputting corresponding prompt instructions.

### Embodiment Four:

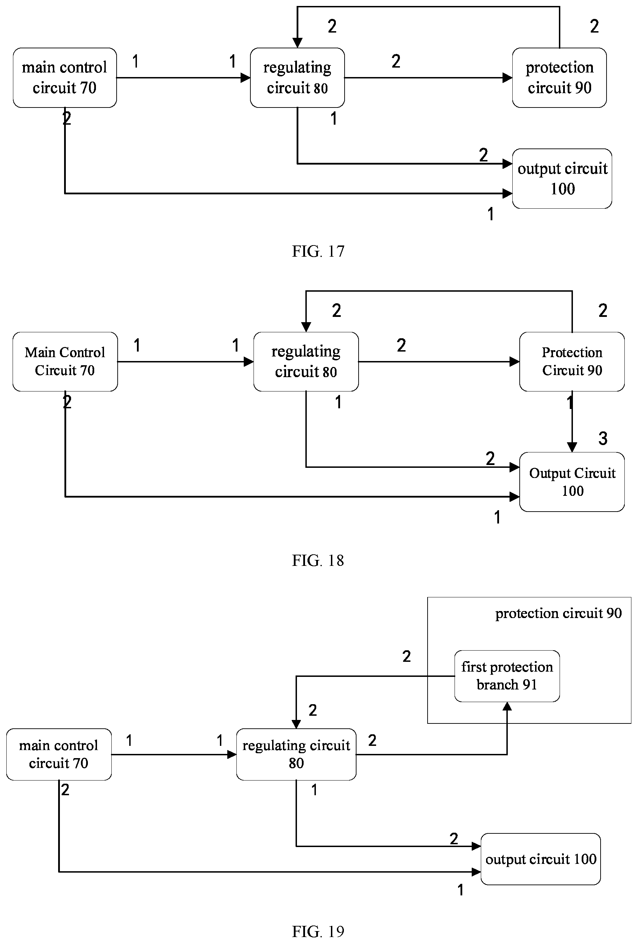

Embodiment Four of the present invention involves an output control circuit. As shown in FIG. 17, the circuit includes a protection circuit 90, an output circuit 100, a regulating circuit 80 with a MOSFET, and a main control circuit 70 with a main control chip.

The first output end of the main control circuit 70 is connected to the first input end of the regulating circuit 80 to transmit a PWM signal. The second output end of the main control circuit 70 is connected to the first input end of the output circuit 100. The first output end of the regulating circuit 80 is connected to the second input end of the output circuit 100, and the second output end of the regulating circuit 80 is connected to the input end of the protection circuit 90. The second output end of the protection circuit 90 is connected to the second input end of the regulating circuit 80.

In this case, a protection circuit 90 is set between the input and output ends of the regulating circuit 80 containing the MOSFET to reduce the load current generated by the junction capacitance of the MOSFET during the instant when the PWM signal from the main control circuit 70 is turned off. This can achieve a reduction/elimination of negative pressure and overshoot spikes in the output of the regulating circuit 80 containing the MOSFET. This makes the overall control circuit more stable and improves the safety of the downstream circuits of the control circuit.

Further, as shown in FIG. 18, the first output end of the protection circuit 90 is connected to the third input end of the output circuit 100. Directly connecting the output end of the protection circuit 90 to the output circuit 100 ensures the stable operation of the protection circuit 90 and enhances the reliability of the circuit.

Further, as shown in FIG. 19, the protection circuit 90 includes a first protection branch 91. The second output end of the MOSFET serves as the second output end of the regulating circuit 80 and is connected to the input end of the first protection branch 91. The output end of the first protection branch 91 serves as the second output end of the protection circuit 90 and is connected to the second input end of the regulating circuit 80. The first protection branch 91 includes a first capacitor C11, one end of which serves as the input end of the first protection branch 91, and the other end serves as the output end of the first protection branch 91.

In this case, the first protection branch 91 directly protects the regulating circuit 80. The first capacitor C11 in the first protection branch 91 isolates the input and output of the regulating circuit 80, ensuring its operation. The first capacitor C11 can also store energy that appears during the operation of the regulating circuit 80 due to the jitter of the PWM signal, reducing the negative pressure generated by the regulating circuit 80 due to the jitter of the PWM signal and smoothing out spikes.

In one example, the output end of the first protection branch 91 serves as the second output end of the protection circuit 90 and is connected to the second input end of the regulating circuit 80.

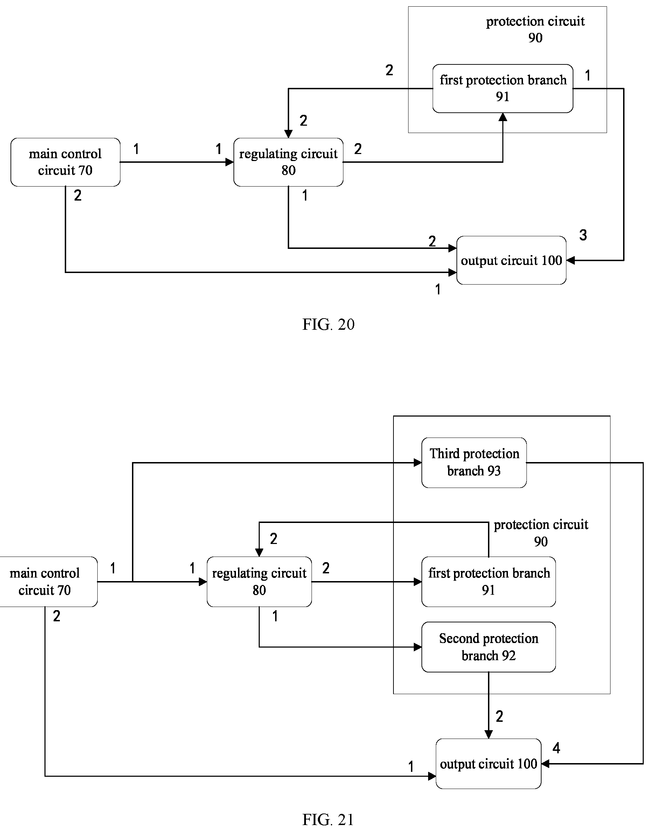

In another example, the first protection branch 91 of the protection circuit 90 is connected to the output circuit 100. Specifically, as shown in FIG. 20, the output end of the first protection branch 91 also serves as the first output end of the protection circuit 90 and is connected to the third input end of the output circuit 100. The connection between the first protection branch 91 and the output circuit 100 helps maintain the circuit from the protection circuit to the output circuit, enhancing the stability of the circuit operation.

Further, as shown in FIG. 21, the protection circuit 90 also includes a second protection branch 92 with a first protection resistor R38. The second protection branch 92 is connected in series between the first output end of the regulating circuit 80 and the second input end of the output circuit 100. One end of the first protection resistor R38 is connected to the first output end of the regulating circuit 80, and the other end is connected to the second input end of the output circuit 100.

In this case, a first protection resistor R38 is added to the direct connection branch between the regulating circuit and the output circuit. The presence of the first protection resistor R38 reduces the impact of the output jitter of the regulating circuit 80 on the overall circuit.

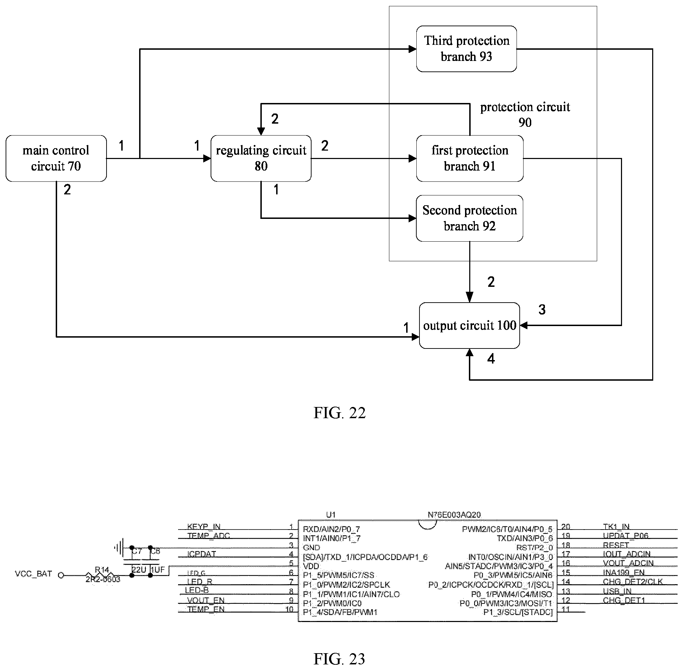

Further, as shown in FIG. 21 and FIG. 22, the protection circuit 90 also includes a third protection branch 93, which includes a second protection resistor R40. One end of the second protection resistor R40 is connected to both the input end of the regulating circuit 80 and the first output end of the main control circuit 70, and the other end is connected to the fourth input end of the output circuit, which is different from the first input end.

In this case, a third protection branch is set between the first output end of the main control circuit 70 and a non-first input end of the output circuit. By setting a high-value second protection resistor, short-circuiting of the entire control circuit due to a fault in the regulating circuit 80 (e.g., breakdown of the regulating circuit 80) is avoided.

Further, the protection circuit 90 with the second protection branch 92 also includes a third protection branch 93. The third protection branch 93 includes a second protection resistor R40, one end of which is connected to both the input end of the regulating circuit 80 and the first output end of the main control circuit 70, and the other end is connected to the third input end of the output circuit 100 and the output end of the first protection resistor R38.

Further, the protection circuit 90 also includes a fourth protection branch 94, which includes a current detection chip and a surface-mount MOSFET. The input end of the current detection chip is connected to the first output end of the regulating circuit 80, and the output end of the current detection chip is connected to the IOUT_ADCIN of the main control chip. The feedback pin and ground pin of the current detection chip are connected to the drain of the surface-mount MOSFET, with the source of the surface-mount MOSFET grounded.

In this case, the current detection chip directly detects the current or voltage output by the regulating circuit 80 to calculate the current output by the second output end of the regulating circuit 80, thereby determining whether the output of the regulating circuit 80 is in a negative pressure state.

Further, the second output end of the main control circuit 70 is connected to the first input end of the output circuit 100, and the second output end of the main control circuit 70 outputs another PWM signal. In this case, the output circuit 100 can be set with a new input end to meet the needs of multiple PWM signal oscillations.

Further, the second output end of the main control circuit is connected to one end of a second capacitor C17, with the other end of the second capacitor C17 grounded. The grounding setting of the second capacitor C17 enhances the anti-interference capability of the first input end of the output circuit 100 for the PWM signal.

### Embodiment Five:

Embodiment Five of the present invention provides an output control circuit. This embodiment is a specific example of the circuit structure in Embodiment Four, and the specific details of the implementation in Embodiment Four remain valid in this embodiment and will not be repeated here.

This embodiment involves a control circuit for an electrical circuit, including a protection circuit, an output circuit, a regulating circuit with a MOSFET, and a main control circuit with a main control chip.

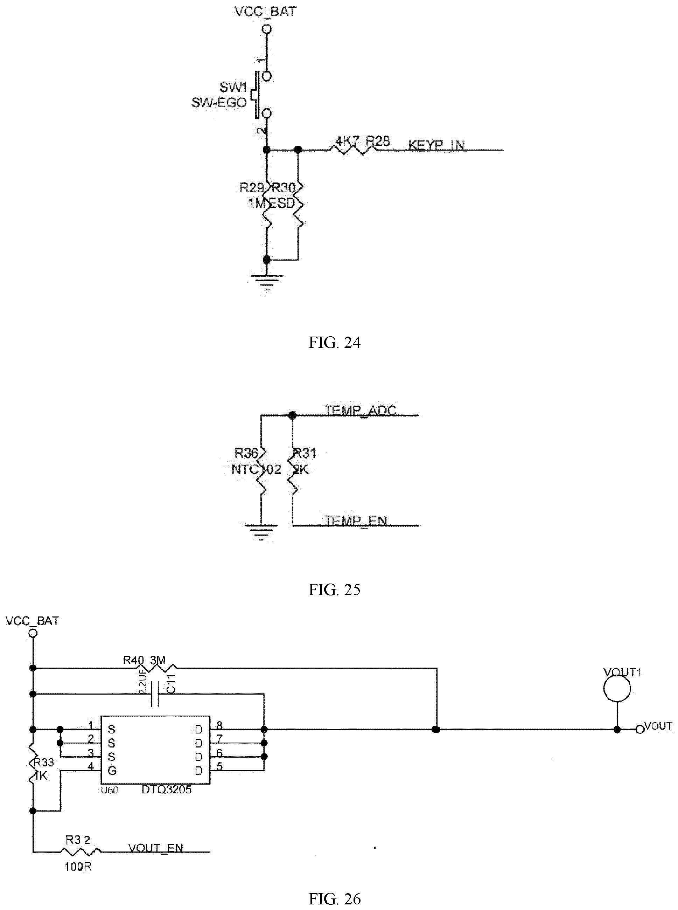

In one example, the main control circuit includes a main control chip U1, a switch circuit, and a temperature control circuit. The main control chip uses an N76E003AQ20 model chip. The switch circuit includes a button SW1 and a static resistor R30ESD. The temperature control circuit includes a thermistor. Specifically, the circuit diagram of the main control chip U1 is shown in FIG. 23, the circuit diagram of the switch circuit is shown in FIG. 24, and the circuit diagram of the temperature control circuit is shown in FIG. 25:

The power supply is VCC_BAT connected in series with pin 1 of the button SW1. One branch of pin 2 of the button SW1 is connected to pin 1 of the main control chip U1 (KEYP_IN) through a fixed resistor R28. Another branch of pin 2 of the button SW1 is connected to a fixed resistor R29 and then grounded. Pin 2 of the button SW1 is also connected to a static resistor R30 and then grounded. The power supply supplies power to the main control chip U1 in the main control circuit through the switch circuit. Pin 2 of the main control chip U1 (TEMP_ADC) is connected to the temperature control circuit through a fixed resistor R31 and then connected to pin 10 of the main control chip U1 (TEMP_EN). The TEMP_ADC is also connected to a thermistor R36 and then grounded.

In one example, as shown in FIG. 26, the regulating circuit includes a MOSFET U60 with a model number DTQ3205. Pins 1, 2, and 3 of the MOSFET are all connected to the power supply VCC_BAT. Pin 4 of the MOSFET is connected in series with a fixed resistor R32 and then connected to pin 9 of the main control circuit U1 (VOUT _EN), which means that pin 9 of the main control chip U1 serves as the first output end of the main control circuit. The first output end of the main control circuit is connected to the power supply VCC_BAT through a fixed resistor R33.

The protection circuit includes a first protection branch, which includes a first capacitor C11. The left end of the first capacitor C11 is connected to the input end of the MOSFET U60 (i.e., pins 1, 2, and 3), which is also the power supply end of the MOSFET U60 (connected to VCC_BAT). The right end of the first capacitor C11 is directly connected to the output circuit VOUT.

The protection circuit includes a third protection branch, which includes a second protection resistor R40. The left end of the second protection resistor R40 is connected to the input end of the MOSFET U60 (i.e., pins 1, 2, and 3), which is also the power supply end of the MOSFET U60 (connected to VCC_BAT). The right end of the second protection resistor R40 is directly connected to the output circuit VOUT.

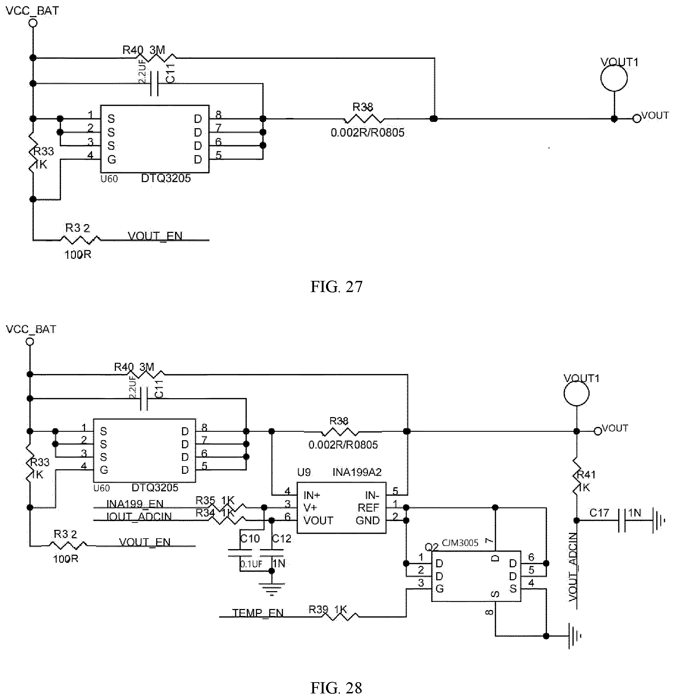

In one example, as shown in FIG. 27, the protection circuit also includes a second protection branch, which includes a first protection resistor R38. The left end of the first protection resistor R38 is connected to the output end of the regulating circuit (at least one of pins 5, 6, 7, or 8 of U60), and the right end of the first protection resistor R38 is connected to the input end of the output circuit.

In one example, when the protection circuit includes the first, second, and third protection branches simultaneously, the connection point of the third protection branch to the output circuit is located to the right of the first protection resistor R38 in the second protection branch, and the connection point of the first capacitor in the first protection branch to the regulating circuit is located to the left of the first protection resistor R38 in the second protection branch.

In one example, as shown in FIG. 28, the protection circuit also includes a fourth protection branch, which includes a current detection chip U9 with a model number INA199A2 and a surface-mount MOSFET Q2 with a model number CJM3005. The input end IN+ of the current detection chip U9 is connected to the second output end of the regulating circuit (the connection point is located to the left of the first protection resistor R38). The input end IN- of the current detection chip U9 is connected to the second, third, or fourth input end of the output circuit (the connection point is located to the right of the first protection resistor R38) (the second, third, and fourth input ends are merged into one point in FIG. 28). This calculates the current flowing through the first protection resistor R38 in the second protection branch. The ground end GND of the current detection chip U9 is connected to the D end of the surface-mount MOSFET Q2. The G end of the surface-mount MOSFET Q2 is connected to pin 10 of the main control chip U1 (TEMP_EN), and the S end of the surface-mount MOSFET Q2 is grounded.

In one example, as shown in FIG. 22, the output circuit includes a first input end, a second input end, a third input end, and a fourth input end. As shown in FIG. 28, the first input end is connected to pin 9 of the main control chip U1 (VOUT_ADCIN). The second, third, and fourth input ends are merged into one point. The first input end of the output circuit is connected to the upper end of a fixed resistor R41. The lower end of the fixed resistor R41 is connected to pin 9 of the main control chip U1 (VOUT_ADCIN) and also connected to one end of a second capacitor C47, with the other end of the second capacitor C17 grounded.

### Embodiment Six:

Embodiment Six of the present invention provides an aerosol generation apparatus, including any one of the output control circuits proposed in Embodiments Four or Five and an aerosol atomization apparatus connected to the output end of the output circuit in the control circuit.

The above descriptions are merely embodiments of the present invention. The specific structures and characteristics commonly known in the solutions are not described in detail here. Ordinary technical personnel in the field are aware of all the common technical knowledge in the field of invention before the application date or priority date, and are capable of applying conventional experimental means on that date. Ordinary technical personnel in the field can improve and implement this solution based on the inspiration given in this application. Some typical common structures or common methods should not become obstacles for ordinary technical personnel in the field to implement this application. It should be pointed out that for those skilled in the art, several transformations and improvements can still be made without departing from the structure of the present invention. These should also be considered within the scope of protection of the present invention. They will not affect the implementation effect and patent practicality of the present invention. The scope of protection required by the present application should be based on the content of its claims, and the specific implementation methods and other records in the description can be used to interpret the content of the claims.

## Claims

1. An aerosol generation apparatus, **characterized in that**, comprising a power supply circuit, a switch circuit, a control circuit, and a detection circuit, an output end of the power supply circuit is connected to a first input end of the control circuit for supplying power to the control circuit through the power supply circuit; an output end of the detection circuit is connected to a second input end of the control circuit, the detection circuit is configured to generate an electrical signal based on a detected connection status of an atomizer and output the electrical signal to the control circuit.

2. The aerosol generation apparatus according to claim 1, **characterized in that**, the power supply circuit comprises a battery and a charging circuit, the charging circuit is configured to supply power to the battery, the charging circuit comprises at least one of USB, Type-C, charging terminal, or wireless charging.

3. The aerosol generation apparatus according to claim 1, **characterized in that**, the power supply circuit comprises a battery and a protection circuit, the protection circuit is configured to protect the battery, one end of the protection circuit is connected to the battery, and an other end is connected to the control circuit.

4. The aerosol generation apparatus according to claim 1, **characterized in that**, the detection circuit comprises a plug-in detection circuit, the plug-in detection circuit is configured to detect a connection status of the atomizer, the plug-in detection circuit comprises a detection resistor R31.

5. The aerosol generation apparatus according to claim 4, **characterized in that**, the detection circuit further comprises a resistance detection circuit, the resistance detection circuit comprises a MOSFET Q2 and a voltage divider resistor R25, the MOSFET Q2 is activated by a control signal from the control circuit to start the resistance detection circuit, the voltage divider resistor R25 is connected in series with the atomizer.

6. The aerosol generation apparatus according to claim 1, **characterized in that**, the aerosol generation apparatus further comprises a charging interface insertion detection circuit, the charging interface insertion detection circuit comprises a diode D1.

7. The aerosol generation apparatus according to claim 1, **characterized in that**, the aerosol generation apparatus further comprises a load enhancement circuit, the load enhancement circuit comprises a MOSFET Q1.

8. The aerosol generation apparatus according to claim 1, **characterized in that**, the aerosol generation apparatus further comprises a temperature control circuit, the temperature control circuit is configured to monitor the temperature of a circuit board and generate a temperature control signal output to the control circuit.

9. The aerosol generation apparatus according to claim 1, **characterized in that**, the aerosol generation apparatus further comprises a prompt circuit, an input end of the prompt circuit is connected to an output end of the control circuit, for indicating a working or connection status of the atomizer based on a control signal outputted from the control circuit.

10. The aerosol generation apparatus according to claim 1, **characterized in that**, the control circuit comprises a main control chip, the detection circuit is integrated in the main control chip.

11. An output control circuit, **characterized in that**, comprising: a protection circuit, an output circuit, a regulating circuit with a MOSFET, and a main control circuit with a main control chip;
a first input end of the main control circuit is connected to a first input end of the regulating circuit and transmits a PWM signal; a second output end of the main control circuit is connected to a first input end of the output circuit;
a first output end of the regulating circuit is connected to a second input end of the output circuit, a second output end of the regulating circuit is connected to an input end of the protection circuit; a second output end of the protection circuit is connected to a second input end of the regulating circuit.

12. The output control circuit according to claim 11, **characterized in that**, a first output end of the protection circuit is connected to a third input end of the output circuit.

13. The output control circuit according to claim 11, **characterized in that**, the regulating circuit comprises the MOSFET, the protection circuit comprises a first protection branch;
a second output end of the MOSFET serves as the second output end of the regulating circuit and is connected to an input end of the first protection branch; an output end of the first protection branch serves as the second output end of the protection circuit and is connected to a second input end of the MOSFET, which serves as the second input end of the regulating circuit; the first protection branch comprises a first capacitor, one end of the first capacitor serves as the input end of the first protection branch, and an other end of the first capacitor serves as the output end of the first protection branch.

14. The output control circuit according to claim 12, **characterized in that**, the protection circuit comprises a second protection branch with a first protection resistor; one end of the first protection resistor is connected to the second output end of the regulating circuit, and the other end is connected to the second input end of the output circuit.

15. The output control circuit according to claim 13, **characterized in that**, the protection circuit further comprises a third protection branch, the third protection branch comprises a second protection resistor, one end of the second protection resistor is connected to an input end of the regulating circuit and an output end of the main control circuit, and an other end of the second protection resistor is connected to a fourth input end of the output circuit, the fourth input end is different from the first input end.

16. The output control circuit according to claim 11, **characterized in that**, the protection circuit further comprises a fourth protection branch, the fourth protection branch comprises a current detection chip and a surface-mount MOSFET, an input end of the current detection chip is connected to the first output end of the regulating circuit, an output end of the current detection chip is connected to one end of the surface-mount MOSFET, and an other end of the surface-mount MOSFET is grounded.

17. The output control circuit according to claim 11, **characterized in that**, the second output end of the main control circuit is connected to the first input end of the output circuit, the second output end of the main control circuit outputs another PWM signal.

18. The output control circuit according to claim 17, **characterized in that**, the second output end of the main control circuit is connected to one end of a second capacitor, another end of the second capacitor is grounded.

19. The output control circuit according to claim 14, **characterized in that**, the protection circuit further comprises a third protection branch, the third protection branch comprises a second protection resistor, one end of the second protection resistor is connected to an input end of the regulating circuit and the first input end of the main control circuit, and an other end of the second protection resistor is connected to a fourth input end of the output circuit and an output end of the first protection resistor.

20. An aerosol generation apparatus, **characterized in that**, comprising an output control circuit according to any one of claims 11 to 19 and an atomizing apparatus connected to an output end of the output circuit.
